# EUROPEAN PATENT SPECIFICATION

(11) **EP 4 624 217 B1**
(45) Date of publication and mention of the grant of the patent: **07.10.2026**
(21) Application number: 25165270.7
(22) Date of filing: 21.03.2025
(51) Int. Cl.: B60K 28/06, G01N 33/497, A61B 5/08

(54) **METHOD FOR CONTINUOUSLY MONITORING THE SOBRIETY STATUS OF A DRIVER**
VERFAHREN ZUR KONTINUIERLICHEN ÜBERWACHUNG DES NÜCHTERNHEITZUSTANDES EINES FAHRERS
PROCÉDÉ DE SURVEILLANCE CONTINUE DE L'ÉTAT DE SOBRIÉTÉ D'UN CONDUCTEUR

(30) Priority: 25.03.2024 IT 202400006568
(43) Date of publication of application: 01.10.2025
(73) Proprietor: S.D.S.Srl Safety Driving Solutions Srl, 19124 La Spezia (IT)
(72) Inventor: FAILLA, RICCARDO, 19124 LA SPEZIA (IT); TERENZIANI, MASSIMO, 19124 LA SPEZIA (IT); GENTILE, VALENTINA, 19124 LA SPEZIA (IT)
(74) Representative: Leotta, Antonio

(56) References cited:
- DE-A1- 102022 121 613
- US-A1- 2018 235 581
- US-A1- 2022 024 308
- US-A1- 2024 017 610

## Description

### FIELD OF THE INVENTION

**.** The object of the present invention is a method for continuously monitoring, i.e. during an entire period of driving, the sobriety status of a driver.

### PRIOR ART

**.** Ethyl alcohol detectors are generally known to be able to detect a value indicative of the concentration of ethyl alcohol (ethanol) in a person's exhalations. Recently, automatic safety solutions have emerged, installed on motor vehicles such as cars and/or commercial vehicles and/or public transport vehicles which are capable of decoupling the control of the vehicle from the driver in case of detection of excessive blood alcohol levels. In these known solutions, a control unit is associated with an ethyl alcohol detector and the vehicle's driving enabling devices. Typically, when the electrical panel is switched on, the driving of the vehicle is temporarily inhibited by the control unit and a detection using the ethyl alcohol detector is requested: if the concentration of ethyl alcohol detected is greater than a threshold value, the control unit keeps the vehicle driving enable locked, while if it is lower, it deactivates the lock and allows the driving of the vehicle.

**.** However, these known systems can encounter many false positives as the exhalations of the person blowing in the ethyl alcohol detector in addition to the alcohol present in the lungs also detect the alcohol present in the mouth due to the use of mouthwashes, due to the presence of alcohol in the environment or due to other reasons, so the concentration detected does not correspond to the alveolar concentration and can also be much higher than the latter. To overcome this problem, US 2022/0024308 proposes a control system that provides for the acquisition by ethyl alcohol detector of a plurality of subsequent measurements of the concentration of alcohol in the breath to calculate a rate of change of the concentration of ethyl alcohol and verify that this is compatible with a dissipation rate of alcohol typical of humans. The proposed method is quite effective in identifying false positives and therefore makes the measurement of the ethyl alcohol detector more reliable, but there are still limits connected to this solution. In fact, the solution described above requires longer execution times as more measurements need to be performed at certain time intervals. Since it is necessary to wait for the outcome of the check before consent is given to start the vehicle, there is a considerable loss of time for those who have to drive. In addition, since the measurement is made by means of an ethyl alcohol detector, it is not possible to perform measurements while the driver is driving, so there can be no control during the journey, unless the driver stops to perform measurements with consequent inconvenience and loss of time.

**.** DE102022121613 describes another device and method which are intended to determine information relating to the breath, in particular the alcohol content in the breath, of an occupant of a vehicle. The system involves the use of both cameras and sensors to allow to overcome the problem created by the presence of several occupants in the passenger compartment of the vehicle that affects the accuracy of the measurement. However, even this document does not solve the problem of monitoring the driver's status of alteration effectively throughout the journey.

### SUMMARY OF THE INVENTION

**.** It is an object of the present invention to overcome the drawbacks lamented with reference to the state of the art and to provide a solution to the above-mentioned needs.

**.** This and other objects are achieved at least in part with a method according to claim 1, as well as with an ethyl alcohol detector assembly according to claim 6.

**.** Some advantageous embodiments are the subject-matter of the dependent claims.

**.** According to one aspect of the invention, a method comprises the steps defined in claim 1.

. The method may be executable by a computer program (computer).

. The steps of storing in memory, and/or processing and/or determining the sobriety status of the driver can be performed automatically remotely.

. The method is usable to decouple the control of a vehicle from the driver.

. According to another aspect of the invention, an operational assembly comprises the features defined in claim 5.

### BRIEF DESCRIPTION OF THE FIGURES

. Further features and advantages of the invention will appear from the following description of embodiments, given by way of non-limiting example, with reference to the accompanying figures, wherein:
- figure 1 is a diagram illustrating some steps of a method, as well as an assembly, according to an embodiment;
- figure 2 is a diagram illustrating the content of a memory according to an embodiment,

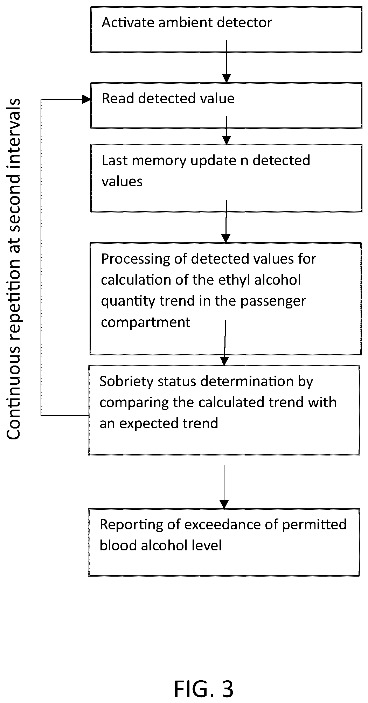

- figure 3 is a flow chart illustrating some possible steps of a method, according to an embodiment.

### DETAILED DESCRIPTION OF SOME PREFERRED EMBODIMENTS

**.** In accordance with a general embodiment, a method for continuously monitoring a sobriety status of a driver is provided.

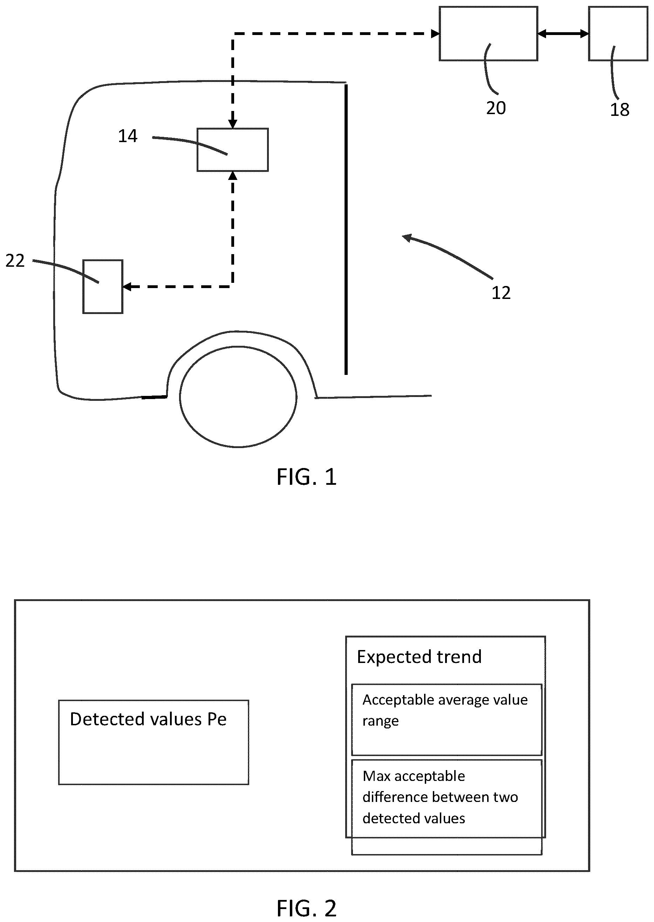

**.** In the example illustrated in figure 1, reference is made to the case of particular interest in which the method is envisaged to decouple the control of a vehicle 12 from its driver in the event of an assessment of excessive blood alcohol content. The method of this disclosure will be illustrated with reference to this feature of particular interest, typical in the field of ethyl alcohol detectors, but it is understood that what will be said also applies in the case in which the method is provided even only to report excessive blood alcohol level.

**.** Therefore, the method for continuously monitoring the sobriety status of a driver comprises the steps of activating an ambient ethyl alcohol detector, 14, located in the passenger compartment of a vehicle 12, continuously detecting the amount of ethyl alcohol present throughout the time the vehicle is in operation, processing the detected values to determine the sobriety status of the driver equally continuously.

**.** The ambient ethyl alcohol detector 14 may be disposed at any location within the passenger compartment of the vehicle 12.

**.** Advantageously, the ethyl alcohol detector 14 is able to measure the amount of ethyl alcohol present in the passenger compartment with a certain sampling frequency, for example tens of measurements per second, however the detector 14 is interrogated to obtain detected values, Pe, at time intervals preferably of a few seconds.

**.** The method comprises the further step of storing in a memory in readout order, the last n detected values Pe, such that the stored detected values cover a time frame of the magnitude of minutes. The storage preferably takes place in a vector, in which the detected values are stored in sequence thereby containing information on the detection time sequence of the detected values Pe.

**.** The method further comprises the step of processing the detected values Pe present in the memory to obtain information on the evolution over time of the ethyl alcohol content in the passenger compartment.

**.** The method further comprises a step of determining the sobriety status of the driver by comparing the previously obtained information on the evolution over time of the ethyl alcohol content in the passenger compartment with an expected evolution.

**.** Advantageously, the expected evolution is continuously updated as it is a function of historical data relating to the values detected Pe.

**.** According to the method of the invention the steps of processing the detected values Pe and determining the sobriety state of the driver are continuously repeated at each acquisition of a new detected value for as long as the ambient detector 14 remains active, i.e. advantageously for as long as the vehicle 12 is in operation. Advantageously, it is intended that the vehicle 12 is in operation when its electrical panel is switched on. Alternatively, it may be understood that the vehicle 12 is in operation even when the relative electrical panel is not switched on but is still powered by a battery.

**.** The step of determining the sobriety status of the driver is repeated every time a new detected value Pe is acquired and only when the outcome of the step is that the driver is not sober is a step of sending a report to that effect performed. For example, a report is transmitted to the driver of the vehicle or other people interested in knowing that an allowed blood alcohol level has been exceeded. Thanks to this method, it is possible to warn the driver of the vehicle or other concerned people, such as those responsible for a fleet of company vehicles, that the driver cannot continue driving the vehicle.

**.** Furthermore, according to an embodiment, the method provides a step of decoupling the control of the vehicle from the driver when, following the step of determining the sobriety status of a driver, a condition of exceeding the permitted blood alcohol level is the result.

**.** The method of the invention takes into account that the dissipation rate of ethanol in the alveolar component of the breath has a known trend, but is based on statistical values, time series and self-learning algorithms to allow discrimination not only between the alveolar alcohol component and other alcohol components in the breath, but also with other ambient disturbance factors such as sanitizing gels, perfumers or more.

**.** To do this, instead of being carried out with a breath ethyl alcohol detector as in all prior art systems, the detection of ethyl alcohol is carried out by means of an ambient ethyl alcohol detector 14 placed in the passenger compartment of the vehicle 12. This allows for a continuous detection as the driver does not have to stop to perform the detections as is typically the case with ethyl alcohol detectors.

**.** In accordance with a possible operating mode, the step of storing the detected values in a memory is carried out remotely. In accordance with a possible operating mode, the processing step is carried out automatically remotely. In accordance with a possible operating mode, the step of determining the sobriety status of a driver is carried out automatically remotely. The term "remote" is here also intended to indicate a connection with a remote operating unit, for example of the "cloud" type.

**.** In accordance with a possible operating mode, the step of transmitting control signals can be performed by a data processing unit 20 that remotely controls control means 22 of the vehicle 12, such as for example a control unit of the vehicle that in turn controls signalling devices of the vehicle and/or one or more actuators of the vehicle.

**.** In accordance with a possible operating mode, the step of transmitting control signals aimed at decoupling the control of the vehicle from the driver is carried out when the vehicle is in a parked condition and the step of transmitting control signals aimed at decoupling the control of the vehicle from the driver comprises blocking the vehicle by preventing it from being started.

**.** The decoupling of the vehicle control from the driver can be accomplished in several ways, and does not necessarily comprise blocking the vehicle by preventing it from being started.

**.** In accordance with a possible operating mode, the step of transmitting control signals aimed at decoupling the control of the vehicle from the driver comprises issuing a signal aimed at informing the driver and/or instructing the driver to avoid and/or stop driving immediately. Such signaling may be acoustic, luminous, a text message, and/or the like.

**.** In accordance with a possible operating mode, the step of transmitting control signals aimed at decoupling the control of the vehicle from the driver comprises deactivating the dashboard of the vehicle for a certain duration of time.

**.** In accordance with a possible operating mode, the step of transmitting control signals aimed at decoupling the control of the vehicle from the driver is performed even if the vehicle is in forward moving condition.

**.** In accordance with a possible operating mode, the method further comprises the step of performing a calibration. This step may comprise the step of setting said detected value in the step of detection as a reference value, for example equal to zero, for the subsequent steps of processing and performing at least one control.

**.** According to a possible operating mode, the processed information comprises information on the average value of the detected quantity, e.g. calibrated on a calculated and/or detected value, and wherein the expected trend comprises a threshold range Rmin-Rmax, and wherein the step of performing at least one control comprises verifying that the average value of the detected quantity belongs to the threshold range.

**.** In accordance with a possible operating mode, the method comprises storing the detected values in a vector so that the sequence of the stored values provides information on the detection sequence; and verifying, by proceeding backwards in the vector of the stored values, that the deviation between the last detected value and the previously detected values is less than or equal to a deviation threshold value M, until a stored value is identified that is greater than the deviation threshold value M; and neglecting, for example by deleting them from the vector, the values stored previously to said stored value that is greater than the deviation threshold value M. At this point, the method may comprise the step of reallocating, for example by translating them backwards in the vector and/or by storing them in another vector, the values stored in the vector. In this way, the computational efficiency is improved because too distant values that are probably indicative of a status of ethanol quantity not of interest because too low are discarded.

**.** Preferably, the detections are spaced apart by a number of seconds. In other words, even if the ambient detector 14 has a higher sampling frequency, it is interrogated at time intervals of the magnitude of seconds, so that between two successive detected values Pe there is a time interval of the magnitude of seconds.

**.** In accordance with a possible operating mode, the step of detecting is repeated at time intervals having a variable duration depending on the outcome of said at least one control. Thereby, the sampling frequency of the alcohol amount values indicative of the driver's blood alcohol level is varied by the control system on the basis of the measured values and/or their evolution over time.

**.** The sampling frequency of the values can be constant.

**.** In accordance with a possible operating mode, the step of detecting is repeated at time intervals of between 1 and 60 seconds, and preferably of a duration of between 5 and 30 seconds. For example, this duration is about 20 seconds.

**.** In accordance with a possible operating mode, the step of detecting is repeated according to the time intervals indicated above for as long as the vehicle is in operation. However, the step of storing in the memory provides that only the last n detected values in order of acquisition time are stored in the memory, and therefore processed. For example, advantageously a quantity n of detected values stored in the memory can be between 10 and 100 so that the steps of processing and determining the sobriety status of the driver are based on a time horizon of the magnitude of minutes.

**.** In accordance with the invention as defined in claim 5, and with reference to the above, an operational assembly 10 for controlling the sobriety status of a driver is provided.

**.** The operational assembly 10 comprises at least one ambient ethyl alcohol detector 14 placed in the passenger compartment of a vehicle for detecting information on the amount of ethyl alcohol in the passenger compartment.

**.** The operational assembly 10 comprises an electronic control system operationally connected to said ambient detector 14, comprising a memory 18 containing information on the expected trend of the amount of ethyl alcohol over time, and a data processing unit 20 for comparing the detected information on the amount of ethyl alcohol with said expected trend of the memory.

**.** The operational assembly 10 further comprises control means 22 operable under the control of the control system and configured to alert about the presence of an excessive blood alcohol level and/or decouple the control of a vehicle 12 to which said ambient detector 14 is associated.

**.** The memory 18 may be in remote operational connection relationship with said at least one ambient detector 14.

**.** The data processing unit 20 is in remote operational connection relationship with said at least one ambient detector 14.

**.** The control means 22 preferably comprise at least a portion thereof suitable for activating signalling means present within the passenger compartment of the vehicle. The control means 22 further preferably comprise at least a portion thereof suitable for performing an action on the vehicle 12, such as for example an electro-mechanical, or hydraulic, or pneumatic actuator capable of decoupling the vehicle from the control of the driver. For example, the assembly may further comprise a vehicle control interface, wherein the control means 22 of the control system is configured to decouple said control interface 16 from the vehicle 12.

### EXAMPLE

. The collection of data to plot the expected trend stored in the memory 18 may be based on experimental data. For example, data may be collected using a test bench that, in a confined environment, simulates with electromechanical means a person's breathing, by inserting ethanol into the circuit, and by detecting the amount of ethanol in the confined environment with an ambient ethyl alcohol detector.

. The data collected may be stored in a database. Some of the parameters that may be stored as expected trend may comprise at least one of the following: (i) an acceptability range of the average value of the amount of alcohol in the passenger compartment calculated over a given time interval, for example 5 or 10 minutes; (ii) the maximum permissible variation of the amount of ethyl alcohol in the passenger compartment between two successive measurements, for example at a distance of 10 or 20 seconds. The values of the aforesaid expected trend parameters can be updated over time on the basis of calculation algorithms which take into account the detected values Pe obtained by applying the method of the invention.

**.** In particular, the large-scale implementation of the method of the invention may lead over time to the realisation of a very populous database in order to make the values of the expected trend parameters more and more reliable.

**.** The stored parameters can be compared with the information processed on the evolution over time of the measured values according to the following steps:

**.** A value is detected every twenty seconds and is transmitted to the data processing unit 20 equipped with the computer program and located on a cloud server in cooperation with the memory 18.

**.** The remote transmission of the information may be by means of remote communication means, for example of a cellular type, integrated in the ambient detector 14. The ambient detector 14 can in turn be interfaced with the vehicle controller 12 and example via Bluetooth communication protocol. According to an embodiment, the remote transmission of the information can take place by means of cellular communication means of a device, for example a smartphone, provided to the driver, with which the ambient detector 14 is interfaced thanks to Bluetooth-type communication means.

**.** In the memory 18, a vector having a capacity of thirty samplings (values) that are stored according to a FIFO queue (first in first out) can be provided, maintaining information on the time sequence of acquisition of the samples.

**.** Each new acquisition of a value (or point Pe) is added to the vector. Is the vector full, then the oldest point is deleted and the detected values (points) can be reallocated.

**.** The acquired value or point Pe is compared backwards with the other values of the vector to evaluate whether there is a stored value P having a deviation greater than or equal to a value M. When a stored value is identified that is greater than the acquired value or point Pe added to the deviation threshold value M, then the values previously stored at said identified value P are neglected, for example by deleting them from the vector.

. In other words, the system calculates the point P less distant from Pe such that: $P - Pand \geq M$ and extracts a portion of the tail of the vector in which the first element is the identified point P and the last element is the acquired point Pe.

**.** From the extracted portion, a decrement vector Δ containing the difference value between two adjacent points of the extracted portion is calculated. In other words, the decrease vector comprises in the first position the difference between the value or point P and its next adjacent, and so on until the last point of the decrease vector comprises the difference between the acquired value Pe and the adjacent value previously acquired.

**.** On the decrement vector Δ at least one of the following controls is performed, and preferably both.

**.** CONTROL A. The average value of all points or values must belong to a certain range Rmin-Rmax, that is: $Rmin \leq \frac{sum \left(Δ\right)}{length \left(Δ\right)} \leq Rmax$ where sum (Δ) denotes the sum of the values or points of the decrement vector and length denotes the number of added values or points.

. CONTROL B. The decrease between adjacent measurements must be within a maximum allowed decrease between adjacent measurements.

. As can be seen, by virtue of the characteristics described above provided disjointly or jointly with one another in particular embodiments, it is possible to meet the needs lamented above, obtaining the aforementioned advantages, and in particular:
- a method for monitoring the sobriety status of a driver is provided capable of carrying out a continuous evaluation of an acceptable blood alcohol level for the entire time of use of the vehicle;
- a single cloud server is allowed to be used to assist and control a plurality of vehicles, allowing the creation of a very populous database that makes evaluations particularly reliable;
- a simple and non-invasive solution is provided that does not involve any commitment for the driver.

**.** A person skilled in the art can make numerous modifications, adaptations and replacement of elements with functionally equivalent elements to the embodiments described above, without however departing from the scope of the appended claims.

## Claims

1. Method for continuously monitoring the sobriety status of a driver, including the steps of:
- activating at least one ambient ethyl alcohol detector (14) installed in the passenger compartment of a vehicle (12) and configured to detect the amount of ethyl alcohol present in the passenger compartment continuously with a certain sampling frequency;
- taking readouts of a detected value (Pe) by said at least one ambient detector (14) at constant time intervals of the magnitude of seconds,
- storing in a memory (18) , in readout order, the last n detected values (Pe), with n such that the stored detected values cover a period of time of the magnitude of minutes;
- processing, on the basis of the detected values (Pe) stored in the memory (18), information on the evolution over time of the ethyl alcohol content in the passenger compartment,
- determining the sobriety status of the driver;
- repeating the steps of processing and determining the sobriety status of the driver every time a new detected value (Pe) is acquired and the memory (18) updated;
the method being **characterized in that** the processing step comprises:
- calculating the difference between the last detected value (Pe) and each of the other detected values stored in the memory (18), and
in the event that at least one of the calculated differences is greater than a predetermined threshold difference,
- calculating the average value of the detected values stored in the memory (18), and
- calculating the maximum difference between two subsequent detected values,
and **in that** determining the sobriety status of the driver is carried out by comparing the evolution over time of the ethyl alcohol content in the passenger compartment with an expected evolution, in which the sobriety status of the driver is verified if said average value and/or maximum difference fall within an admissible range of average values and an admissible decrease range, respectively.

2. Method according to claim 1 wherein the step of activating said ambient detector (14) occurs automatically before starting the vehicle (12) and the subsequent steps of storing in a memory (18), processing, determining the sobriety status of the driver and repeating the processing step, occur automatically as long as the vehicle is moving.

3. Method according to one of the previous claims **characterized in that** the detected values are sent in real time to a remote operating unit where the phases of storing in a memory (18), processing and determining the sobriety status of the driver are carried out, and **in that** the outcome of the phase of determining the sobriety status of the driver is communicated via communication means to a device provided in the passenger compartment of the vehicle and the driver is alerted by said device.

4. Method according to claims 1 and 3, wherein said admissible average difference value range and said admissible decrement range are stored in said remote operating unit and are updated based on historical data of detected readings (Pe) received over time by said remote operating unit.

5. Operational assembly (10) for controlling the sobriety status of a driver, in particular for the management of vehicle fleets, comprising:
- at least one ambient ethyl alcohol detector (14) located inside the passenger compartment of a vehicle, to detect information on the amount of ethyl alcohol present in the passenger compartment;
- an electronic control system operationally connected to said ambient detector, comprising:
- a memory (18) containing information on the expected trend of the ethyl alcohol concentration over time, and
- a data processing unit (20) to compare the information detected on the concentration of ethyl alcohol with said expected trend stored in the memory (18);
said electronic control system being adapted to carry out a method according to any of the previous claims,
said assembly also comprising control means (22) operable under the control of the control system and configured to alert about the presence of an excessive alcohol concentration and/or decouple the control of a vehicle (12) or a device to which said operational assembly can be associated.

6. Assembly (10) according to the previous claim, in which the memory (18) is in remote operational connection relationship with said at least one ambient detector (14), and in which the data processing control unit (20) is also in a remote operating connection relationship with said at least one ambient detector (14).

## Patentansprüche

1. Verfahren zur kontinuierlichen Überwachung des Nüchternheitszustands eines Fahrers, umfassend die folgenden Schritte:
- Aktivieren mindestens eines im Fahrgastraum eines Fahrzeugs (12) installierten Ethylalkohol-Umgebungsdetektors (14), der so ausgelegt ist, dass er die im Fahrgastraum vorhandene Ethylalkoholmenge kontinuierlich mit einer bestimmten Abtastfrequenz erfasst;
- Erfassen von Messwerten (Pe), die von dem mindestens einen Umgebungsdetektor (14) in konstanten Zeitintervallen im Sekundenbereich erfasst werden,
- Speichern der letzten n erfassten Messwerte (Pe) in der Reihenfolge ihrer Erfassung in einem Speicher (18), wobei n so gewählt ist, dass die gespeicherten Messwerte einen Zeitraum im Minutenbereich abdecken;
- Verarbeiten von Informationen über die zeitliche Entwicklung des Ethylalkoholgehalts im Fahrgastraum auf der Grundlage der im Speicher (18) gespeicherten Messwerte (Pe),
- Bestimmen des Nüchternheitszustands des Fahrers;
- Wiederholen der Schritte der Verarbeitung und der Bestimmung des Nüchternheitszustands des Fahrers jedes Mal, wenn ein neuer Messwert (Pe) erfasst und der Speicher (18) aktualisiert wird;
wobei das Verfahren **dadurch gekennzeichnet ist, dass** der Verarbeitungsschritt umfasst:
- Berechnung der Differenz zwischen dem letzten Messwert (Pe) und jedem der anderen im Speicher (18) gespeicherten Messwerte, und für den Fall, dass mindestens eine der berechneten Differenzen größer ist als eine vorbestimmte Schwellenwertdifferenz,
- Berechnung des Durchschnittswerts der im Speicher (18) gespeicherten erfassten Werte und
- Berechnung der maximalen Differenz zwischen zwei aufeinanderfolgenden erfassten Werten,
und dadurch, dass die Bestimmung des Nüchternheitszustands des Fahrers durch den Vergleich des zeitlichen Verlaufs des Ethylalkoholgehalts im Fahrgastraum mit einem erwarteten Verlauf erfolgt, wobei der Nüchternheitszustand des Fahrers bestätigt wird, wenn der genannte Durchschnittswert und/oder die maximale Differenz jeweils innerhalb eines zulässigen Bereichs von Durchschnittswerten bzw. eines zulässigen Abnahmebereichs liegen.

2. Verfahren nach Anspruch 1, wobei der Schritt des Aktivierens des Umgebungsdetektors (14) automatisch vor dem Starten des Fahrzeugs (12) erfolgt und die nachfolgenden Schritte des Speicherns in einem Speicher (18), der Verarbeitung, der Bestimmung des Nüchternheitszustands des Fahrers und der Wiederholung des Verarbeitungsschritts automatisch erfolgen, solange sich das Fahrzeug in Bewegung befindet.

3. Verfahren nach einem der vorstehenden Ansprüche, **dadurch gekennzeichnet, dass** die erfassten Werte in Echtzeit an eine Fernbedienungseinheit gesendet werden, wo die Phasen des Speicherns in einem Speicher (18), der Verarbeitung und der Bestimmung des Nüchternheitszustands des Fahrers durchgeführt werden, und dass das Ergebnis der Phase der Bestimmung des Nüchternheitszustands des Fahrers über Kommunikationsmittel an eine im Fahrgastraum des Fahrzeugs vorgesehene Vorrichtung übermittelt wird und der Fahrer durch diese Vorrichtung gewarnt wird.

4. Verfahren nach den Ansprüchen 1 und 3, wobei der zulässige Bereich für den durchschnittlichen Differenzwert und der zulässige Bereich für die Abnahme in der Fernbedienungseinheit gespeichert sind und auf der Grundlage historischer Daten der von der Fernbedienungseinheit im Laufe der Zeit empfangenen erfassten Messwerte (Pe) aktualisiert werden.

5. Betriebsvorrichtung (10) zur Überwachung des Nüchternheitszustands eines Fahrers, insbesondere für das Management von Fahrzeugflotten, umfassend:
- mindestens einen im Fahrgastraum eines Fahrzeugs angeordneten Ethylalkohol-Umgebungsdetektor (14) zur Erfassung von Informationen über die im Fahrgastraum vorhandene Ethylalkoholmenge;
- ein elektronisches Steuerungssystem, das funktionsmäßig mit dem Umgebungsdetektor verbunden ist und umfasst:
- einen Speicher (18), der Informationen über den erwarteten Verlauf der Ethylalkoholkonzentration im Zeitverlauf enthält, und
- eine Datenverarbeitungseinheit (20) zum Vergleich der erfassten Informationen über die Ethylalkoholkonzentration mit dem im Speicher (18) gespeicherten erwarteten Verlauf;
wobei das elektronische Steuerungssystem dazu ausgelegt ist, ein Verfahren gemäß einem der vorstehenden Ansprüche auszuführen, wobei die Baugruppe ferner Steuermittel (22) umfasst, die unter der Steuerung des Steuerungssystems betreibbar und so konfiguriert sind, dass sie vor dem Vorliegen einer übermäßigen Alkoholkonzentration warnen und/oder die Steuerung eines Fahrzeugs (12) oder einer Vorrichtung, der die Betriebsbaugruppe zugeordnet werden kann, entkoppeln.

6. Baugruppe (10) nach dem vorstehenden Anspruch, wobei der Speicher (18) in einer ferngesteuerten Betriebsverbindung mit dem mindestens einen Umgebungssensor (14) steht und wobei die Datenverarbeitungs-Steuereinheit (20) ebenfalls in einer ferngesteuerten Betriebsverbindung mit dem mindestens einen Umgebungssensor (14) steht.

## Revendications

1. Procédé de surveillance en continu de l'état de sobriété d'un conducteur, comprenant les étapes suivantes :
- activer au moins un détecteur ambiant d'alcool éthylique (14) installé dans l'habitacle d'un véhicule (12) et configuré pour détecter en continu, à une certaine fréquence d'échantillonnage, la quantité d'alcool éthylique présente dans l'habitacle ;
- la lecture, à intervalles de temps constants de l'ordre de quelques secondes, d'une valeur détectée (Pe) par ledit au moins un détecteur ambiant (14),
- le stockage dans une mémoire (18), dans l'ordre de lecture, des n dernières valeurs détectées (Pe), n étant tel que les valeurs détectées stockées couvrent une période de temps de l'ordre de quelques minutes ;
- traiter, sur la base des valeurs détectées (Pe) stockées dans la mémoire (18), des informations relatives à l'évolution dans le temps de la teneur en alcool éthylique dans l'habitacle,
- déterminer l'état de sobriété du conducteur ;
- répéter les étapes de traitement et de détermination de l'état de sobriété du conducteur chaque fois qu'une nouvelle valeur détectée (Pe) est acquise et que la mémoire (18) est mise à jour ;
le procédé étant **caractérisé en ce que** l'étape de traitement comprend :
- le calcul de la différence entre la dernière valeur détectée (Pe) et chacune des autres valeurs détectées stockées dans la mémoire (18), et
dans le cas où au moins l'une des différences calculées est supérieure à un seuil de différence prédéterminé,
- calculer la valeur moyenne des valeurs détectées stockées dans la mémoire (18), et
- calculer la différence maximale entre deux valeurs détectées consécutives,
et **en ce que**
la détermination de l'état de sobriété du conducteur s'effectue en comparant l'évolution dans le temps de la teneur en alcool éthylique dans l'habitacle à une évolution attendue, l'état de sobriété du conducteur étant vérifié si ladite valeur moyenne et/ou ladite différence maximale se situent respectivement dans une plage admissible de valeurs moyennes et dans une plage admissible de diminution.

2. Procédé selon la revendication 1, dans lequel l'étape consistant à activer ledit détecteur d'ambiance (14) se produit automatiquement avant le démarrage du véhicule (12) et les étapes suivantes consistant à stocker dans une mémoire (18), à traiter, à déterminer l'état de sobriété du conducteur et à répéter l'étape de traitement se produisent automatiquement tant que le véhicule est en mouvement.

3. Procédé selon l'une des revendications précédentes, **caractérisé en ce que** les valeurs détectées sont transmises en temps réel à une unité de commande à distance où sont exécutées les phases de stockage dans une mémoire (18), de traitement et de détermination de l'état de sobriété du conducteur, et **en ce que** le résultat de la phase de détermination de l'état de sobriété du conducteur est communiqué par des moyens de communication à un dispositif prévu dans l'habitacle du véhicule et le conducteur est alerté par ledit dispositif.

4. Procédé selon les revendications 1 et 3, dans lequel ladite plage de valeurs de différence moyenne admissibles et ladite plage de décrément admissible sont stockées dans ladite unité de commande à distance et sont mises à jour sur la base des données historiques des mesures détectées (Pe) reçues au fil du temps par ladite unité de commande à distance.

5. Ensemble opérationnel (10) destiné à contrôler l'état de sobriété d'un conducteur, en particulier pour la gestion de flottes de véhicules, comprenant :
- au moins un détecteur ambiant d'alcool éthylique (14) situé à l'intérieur de l'habitacle d'un véhicule, destiné à détecter des informations sur la quantité d'alcool éthylique présente dans l'habitacle ;
- un système de commande électronique relié de manière opérationnelle audit détecteur ambiant, comprenant :
- une mémoire (18) contenant des informations sur l'évolution attendue de la concentration en alcool éthylique au fil du temps, et
- une unité de traitement de données (20) destinée à comparer les informations détectées concernant la concentration en alcool éthylique avec ladite évolution attendue stockée dans la mémoire (18) ;
ledit système de commande électronique étant adapté pour mettre en œuvre un procédé selon l'une quelconque des revendications précédentes,
ledit ensemble comprenant également des moyens de commande (22) pouvant fonctionner sous le contrôle du système de commande et configurés pour signaler la présence d'une concentration excessive en alcool et/ou pour désactiver la commande d'un véhicule (12) ou d'un dispositif auquel ledit ensemble opérationnel peut être associé.

6. Ensemble (10) selon la revendication précédente, dans lequel la mémoire (18) est en liaison opérationnelle à distance avec ledit au moins un détecteur d'environnement (14), et dans lequel l'unité de commande de traitement des données (20) est également en liaison opérationnelle à distance avec ledit au moins un détecteur d'environnement (14).
